# EUROPEAN PATENT APPLICATION

(11) **EP 2 749 654 A1**
(43) Date of publication of application: **02.07.2014**
(21) Application number: 12199784.5
(22) Date of filing: 28.12.2012
(51) Int. Cl.: C12Q 1/68, C12N 15/10

(54) **Method of analysis of composition of nucleic acid mixtures**

(71) Applicant: Max-Planck-Gesellschaft zur Förderung der Wissenschaften e.V., 80539 München (DE)
(72) Inventor: Borodina, Tatiana, Dr., 14195 Berlin (DE); Soldatov, Aleksey, Dr., 14195 Berlin (DE); Lehrach, Hans, Prof., 14129 Berlin (DE)
(74) Representative: Arth, Hans-Lothar

(57) **Abstract**

The present invention relates to a method of analysis of concentrations of nucleic acid components in mixtures containing nucleic acids which allows adjusting the reliability of results individually for each component of the nucleic acid mixture in a highly reproducible manner as well as kits suitable for this method.

## Description

### Field of the invention

When sequencing is used for the analysis of composition of nucleic acid mixtures with a large dynamic range of concentrations of individual components, the reliability of results significantly differs for abundant and rare components. This is a common problem for studying of transcriptomes and for analysis of biodiversity by sequencing of environmental and clinical samples. We suggest a method of analysis which allows adjusting the reliability of results individually for each component of the nucleic acid mixture in a highly reproducible manner: Controllable Oligonucleotide-Based Ratio Adjustment (COBRA). The method is based on using of locus-specific primers to change the relative abundance of individual components of nucleic acid mixture. The method is especially useful for routine analysis of biodiversity and routine expression profiling, f.e., for clinical studies.

### Background of the invention

RNA-Seq is a hypothesis-free approach for studying of transcriptome by sequencing of millions of cDNA fragments. The abundance of cDNA fragments matches the abundance of the corresponding transcript. The obtained sequencing results give a possibility to retrieve information about abundance and structure of transcripts.

RNA-Seq is complicated by two problems:
1. gene expression levels have a huge dynamic range (about 5 orders of magnitude). So, to characterize low-expressed genes it is necessary to oversequence highly expressed ones. The more sequencing reads correspond to a particular transcript, the more reliably its expression level is determined.
2. it is difficult to estimate accurately the expression level of similar transcripts. Similarity of transcripts is a common phenomenon:
   - all genes have two (or more in case of polyploid organisms) homologous copies (alleles);
   - repetitive genomic regions give rise to similar transcripts;
   - individual genes may produce several similar transcripts (splice variants) due to presence of alternative donor- and acceptor-splicing sites.

Only a portion of reads mapped to the similar transcripts may be used for characterisation of expression levels of individual homologues: namely those reads which overlap sites, different between the homologues. Other reads may be used only for characterisation of cumulative expression level.

The most overexpressed RNA in cells is ribosomal RNA, which comprises 90-99% of the total RNA. To avoid the costs related to the sequencing of rRNA, researchers try to remove as much as possible rRNA from the sample before preparation of the sequencing library. Since it is difficult to control which portion of rRNA remains in the sample, in most expression profiling experiments the level of rRNA is not analysed.

To reduce a huge dynamic range of expression levels several protocols for preparation of normalized libraries were suggested. All these protocols are based on the dependence of the rehybridization rate of nucleic acids on concentration. Normalization decreases the representation of highly expressed transcripts in the sequencing library. Normalization has specific problems:
- the normalization effect is limited: after normalization highly expressed genes still produce more sequencing reads than low expressed ones;
- rehybridization rate depends not only on the concentration of the component, but also on its nucleotide sequence;
- highly expressed homologues may suppress a low expressed similar transcripts because of cross-hybridization;
- normalization rate has limited reproducibility and strongly depends on the experimental protocol.

In any case (both for classical RNA-Seq and for normalization-based sequencing) reliability of expression level measurements is different for different transcripts: it strongly depends on expression level and somehow depends on nucleotide sequence. Generally, reliability is higher for highly expressed unique transcripts and lower for low expressed and similar transcripts.

It would be nice if a researcher would have a possibility to choose the reliability of expression level measurements depending on the experimental goal and on properties of the biological system under study. Different transcripts may have different:
- interest to a researcher - for example, expression leves of some genes are important for making a decision in clinical analyses and it is desirable to know them with high accuracy, whereas some others may serve only as general controls - for those high accuracy is not needed,
- distributions in biological system under study. For example, if it is known that the concentration of the first transcript varies within 10% in different biological samples, and of the second - may differ in two times, it makes no sense to measure the concentration of the second transcript with the same accuracy as for the first. The concentration of the first transcript should be measured more accurately than that of the second.

Usually only some portion of RNA-Seq library is sequenced. Concentration of abundant transcripts is determined with excessive reliability, of rare - with insufficient. Sequencing of the rest of the library would improve the reliability of measurement of concentration of rare transcripts. But this would increase the cost of the analysis. It would be more attractive to reduce the number of sequencing reads corresponding to abundant transcripts (analyzed with redundant reliability). In this case the reliability of the analysis of rare transcripts would increase without altering the price of the analysis.

### Description of the invention

The present invention refers to a method of analysis of concentrations of nucleic acid components in mixtures containing nucleic acids, comprising the following steps:
i) providing an original mixture containing nucleic acids;
ii) selection of at least one nucleic acid component of the original mixture for which the abundance should be changed in predefined manner;
iii) creation from original mixture containing nucleic acids a subsequent nucleic acid mixture wherein abundances of components corresponding to the components selected on step ii) are changed in predefined manner using locus-specific oligonucleotides for said components;
iv) analysis of concentrations of at least two components in the subsequent nucleic acid mixture for which relative abundances were changed in predefined manner compared with relative abundances of corresponding components in the original mixture containing nucleic acids.

Thereby it is preferred that components of the original mixture containing nucleic acids and the values of change of abundance are selected on step ii) in such a way, that the dynamic range of concentrations of components under analysis in the subsequent nucleic acid mixture is lower than the dynamic range of concentrations of components under analysis in the original mixture containing nucleic acids or abundances of components under analysis in the subsequent mixture are more suitable for the required accuracy of analysis of concentrations.

Within a preferred embodiment of the method according to the invention the subsequent nucleic acid mixture is selected from the group comprising or consisting of: sequencing library, set of ligated detector oligonucleotides, set of fluorescently labeled molecules, or nucleic acids molecules selected from the original mixture.

Within a preferred embodiment of the method according to the invention analysis of concentration of components in the subsequent nucleic acid mixture on step iv) is performed by sequencing, hybridization with microarray, RT-PCR, digital PCR, digital color-coded barcode technology. Thereby it is preferred that concentrations of components under analysis in original mixture containing nucleic acids are calculated using predefined values of change of abundance of components under analysis.

The inventive methods can be used for routine analyses of biodiversity or expression profiling in medicine, veterinary, agriculture, ecological studies. In regard to the present invention it is preferred that the nucleic acid of the original mixture is selected for expression profiling from the group comprising: RNA, total RNA, mRNA, mtRNA, rRNA, tRNA, dsRNA, small RNA / micro RNA, cDNA; for analysis of biodiversity from the group comprising: RNA or DNA from environmental or clinical sample.

Locus-specific oligonucleotides as used herein are oligonucleotides which hybridize to a unique region in the genome or transcriptome. Their specificity allows selecting a particular region from a complex mixture of nucleic acids for amplification, determination of copy number, genotyping, etc. To participate in these reactions, oligonucleotides may contain not only locus-specific part, but also other regions required for interaction with common amplification primers (constant parts for subsequent amplification), TaqMan probes, hybridization probes (constant part for the hybridization-based selection), probes on microarrays, etc. For bacterial genomes two primers with lengths of 12-20 nucleotides are usually specific enough for targeting a unique locus. For mammalian genomes the length of two primers should be 17-25 nucleotides.

The term "constant parts for subsequent amplification" as used herein refers to external regions of locus-specific oligonucleotides which are not complementary to the target. These regions are the same in a group of locus-specific oligonucleotides. Primers corresponding to these constant parts may be used for PCR amplification.

The term "constant parts for the hybridization-based selection" as used herein refers to particular regions of locus-specific oligonucleotides which are not complementary to the target. These regions are the same in a group of locus-specific oligonucleotides. Primers corresponding to these particular regions may be used for hybridization-based selection. Selector oligonucleotides are used for specific hybridization to target loci and subsequent isolation of hybrids from the nucleic acid mixture.

The subsequent nucleic acid mixture is produced from the original mixture containing nucleic acids. Subsequent nucleic acid mixture may contain components of the original nucleic acid mixture as well as products of interaction of locus-specific oligonucleotides with corresponding components of the original nucleic acid mixture, wherein said products may be extended locus-specific oligonucleotides, ligated sets of locus-specific oligonucleotides, hybrids of locus-specific oligonucleotides with components of the original nucleic acid mixture, sequencing library, set of fluorescently labeled molecules, etc. In the current invention relative abundances of some components in the subsequent nucleic acid mixture may be changed in predefined manner compared with relative abundances of corresponding components in the original mixture containing nucleic acids.

Components of the subsequent nucleic acid mixture corresponding to the components of the original mixture containing nucleic acids may be nucleic acid molecules transferred from the original nucleic acid mixture; products of reaction with locus-specific oligonucleotides or combination of listed variants. A special case is when subsequent nucleic acid mixture comprises special oligonucleotides with codes, wherein each code corresponds to particular locus.

Blocked and functional oligonucleotides for each locus are able to hybridize to the same sequence and compete with each other in the same reaction. Functional oligonucleotides give rise to corresponding nucleic acid components in the subsequent nucleic acid mixture, while blocked oligonucleotides either do not give rise to corresponding nucleic acid components or products originating from blocked oligonucleotides may be separated from the products originating from functional oligonucleotides. The ratio of concentrations of functional to blocked oligonucleotides is equivalent to the predefined value of change of abundance for this locus.3' end modifications (amino group; C3-spacer; biotin; inverted end; terminal dNTP, etc.) may be used to prevent elongation of blocked oligonucleotides in such reactions as primer extension, first-strand synthesis, second-strand synthesis, PCR, gap-filling. 5' end (dephosphorylation; amino group; biotin, etc.) or 3' end (amino group; C3-spacer; biotin; inverted end; terminal ddNTP, etc.) modifications may be used to prevent participation of blocked oligonucleotides in ligation in such reactions as ligation detection reaction, gap-filling, LCR (Ligase Chain Reaction), DANSR (digital analysis of selected regions).
A special marker may be attached to functional and/or blocked oligonucleotides (dUTP; thio-modified oligonucleotides; 5-bromo-2'-deoxyuridine (BrdU); biotin; presence in oligonucleotides of constant parts for subsequent amplification or hybridization-based selection, etc.) to separate from each other hybrids of functional oligonucleotides with target (or products of reaction involving functional oligonucleotides) and the hybrids of blocked oligonucleotides with target (or products of reaction involving blocked oligonucleotides).

Digital PCR is a refinement of conventional polymerase chain reaction methods that can be used to directly quantify nucleic acids. The key difference between dPCR and traditional PCR lies in the method of measuring nucleic acids amounts, with the former being a more precise method than PCR. PCR carries out one reaction per single sample. dPCR also carries out a single reaction within a sample, however the sample is separated into a large number of partitions and the reaction is carried out in each partition individually. This separation allows a more reliable collection and sensitive measurement of nucleic acid amounts.

Digital color-coded barcode technology is based on direct multiplexed measurement of gene expression. The technology uses molecular "barcodes" and single molecule imaging to detect and count hundreds of unique transcripts in a single reaction. Each color-coded barcode is attached to a single target-specific probe corresponding to a gene of interest.

Sequencing library molecules have common flanking sequencing library adaptors, which are used for the clonal amplification of the library molecules in the sequencing machine (illumina, SOLiD).

Unlike to hypothesis-driven approaches when some particular components of the system are analyzed in connection to the studied phenomenon, hypothesis-free approach involves analysis of as much as possible components to find out which of them are related to the studied phenomenon.

### COBRA-approach

We propose to change the concentration of selected components of a cDNA library in a certain number of times before sequencing. This allows to:
- reduce the dynamic range of concentrations of transcripts without loss of information;
- reduce the representation of those transcripts in the library which are analyzed with excess reliability;
- adjust the reliability of concentration measurement of individual transcripts.

To implement this approach we suggest to prepare a sequencing library using locus-specific oligonucleotides. Specific oligonucleotides give a possibility to vary the conditions of library preparation for different transcripts and selectively suppress the efficiency of library preparation for abundant and not-so-important transcripts.

Changing the abundance of sequencing library molecules correspondent to particular transcripts using locus-specific oligonucleotides (Controllable Oligonucleotide-Based Ratio Adjustment -- COBRA) allows to combine the selectivity of microarrays with the accuracy and sensitivity of massive parallel sequencing. As in microarray technologies, kits for preparation of COBRA libraries may include hundreds and thousands sets of locus-specific oligonucleotides. COBRA allows:
- to convert into library molecules only those genes which are interesting to the researcher;
- to convert into library molecules only informative regions of genes: to avoid repeats and other difficult-for-analysis regions;
- to tune the accuracy of the analysis individually for each transcript under study.

### Practical implementation

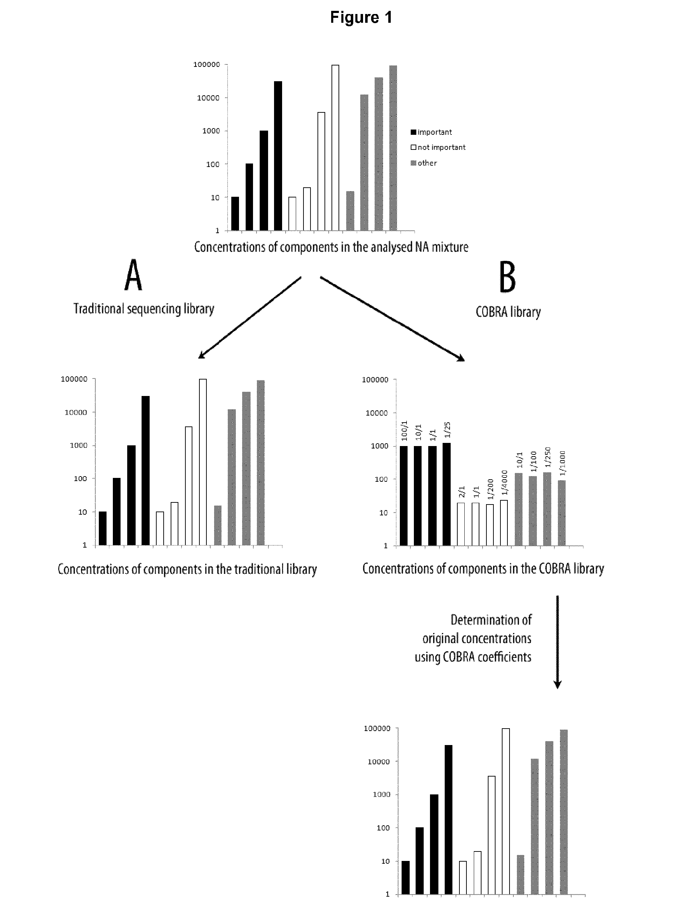

COBRA (Controllable Oligonucleotide-Based Ratio Adjustment) changes the relative abundance (and, as a result, the reliability of analysis) of components under study in a sequencing library in a predictable and reproducible way using locus-specific oligonucleotides (Fig. 1).

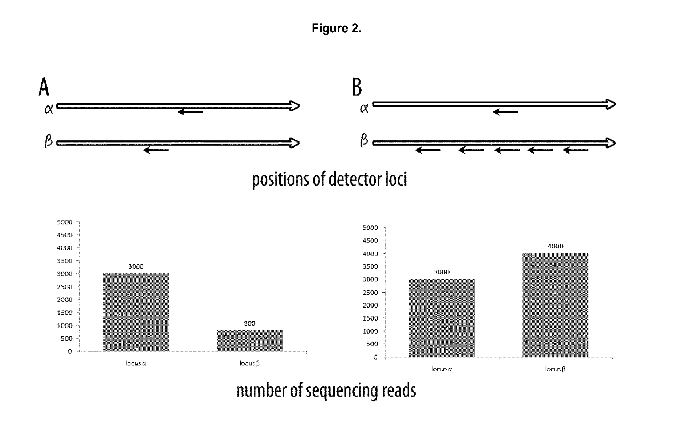

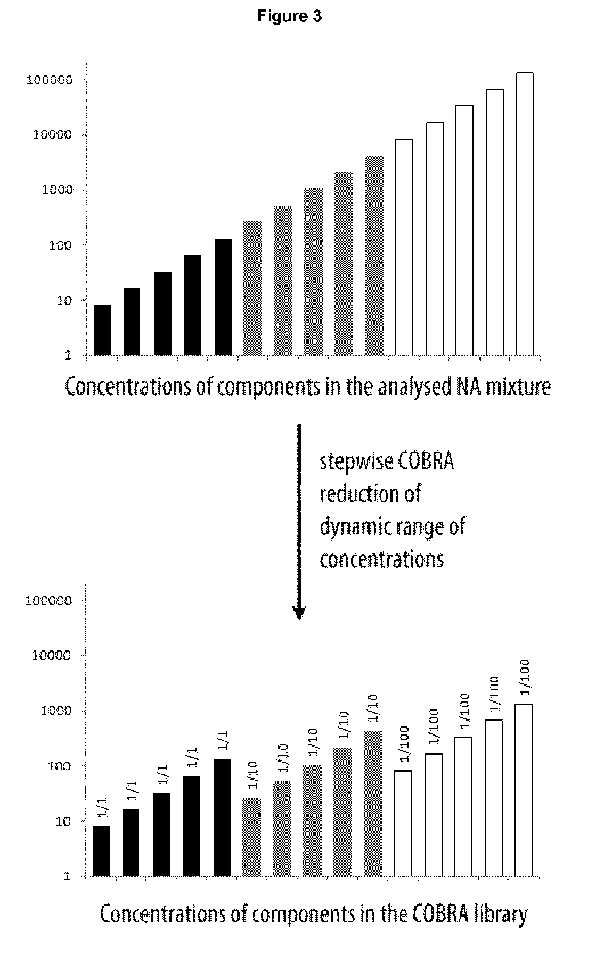

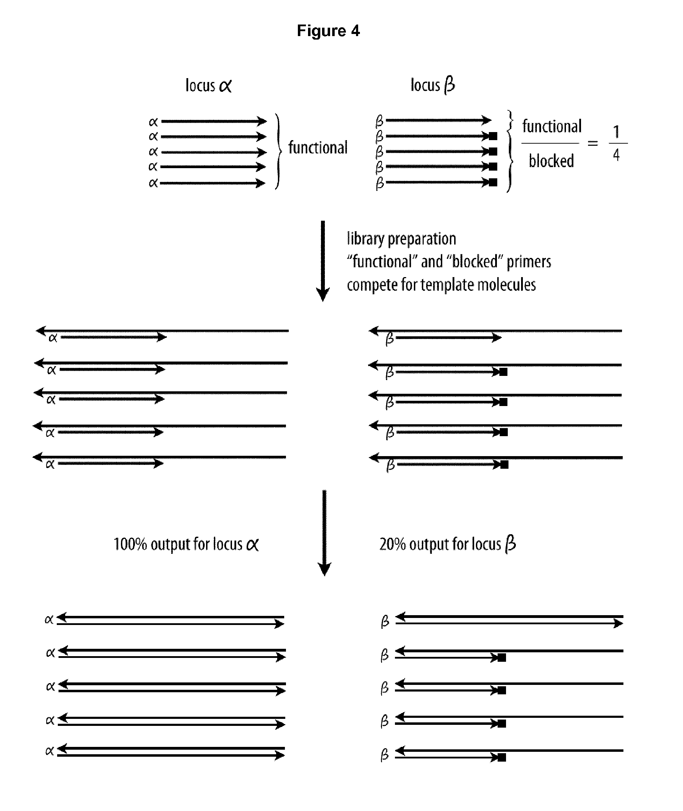

We suggest three methods for regulation of abundance of sequencing library molecules correspondent to different components of nucleic acid mixture:
1) Selection of different number of detectable loci for different components of nucleic acid mixture (Fig. 2).
2) Combining of loci in several groups according to the desirable level of "change of abundance" and using of different library-preparation protocols for different groups (Figure 3, Examples 3-7);
3) Using a mixture of "functional" / "blocked" primers to adjust the level of "change of abundance" individually for each detectable locus (Figure 4, Examples 8-12).

Using these three approaches and their combinations it is possible to offer a variety of protocols for preparation of COBRA sequencing libraries.
Existing methods of preparation of sequencing libraries may be adapted for COBRA. If a method is using locus-specific oligonucleotides, it is possible to use those oligonucleotides for regulation of the content of correspondent sequencing library molecules. If there are no locus-specific oligonucleotides in the protocol, it is possible to introduce them at some stage. For example, when using a standard RNA-Seq protocol locus-specific oligonucleotides may be used for:
- positive selection of RNA molecules;
- negative selection of RNA molecules;
- the first strand synthesis;
- fishing out of certain library molecules by hybridization, etc.

### Number of detectable loci per transcript

The present invention refers further to methods wherein the relative abundance of at least one component under analysis is changed in step iii) by selecting suitable number of loci and correspondent locus-specific oligonucleotides for this component.

If not one but several detectable loci (preferably, located in a way that they do not compete with each other) are selected for a certain transcript, the number of sequencing reads matching this transcript would increase proportionately. This will increase the reliability of concentration measurement of the transcript. This approach is well suited for increasing the reliability of analysis of rare transcripts.

Selection of different number of detectable loci for regulation of abundance of correspondent molecules in sequencing library has certain advantages and disadvantages.

Advantages:
- the method is fully compatible with other COBRA-approaches and library preparation protocols;
- the method allows to easily adjust the abundance in a small range (up to about 10);
- opposite to most other COBRA-approaches, this method does not reduce but increases the abundance.

Disadvantages:
- the number of possible loci may be limited for particular transcript (especially for similar transcripts, where loci should be located in regions, different between the homologues);
- regulation is stepwise;
- the method is not suitable for suppression, only for increasing the abundance;
- the synthesis of new oligonucleotides is required to change the level of regulation.

### Combining loci in "change of abundance" groups

If independent locus-specific adjustment of abundance level is not a goal, and a stepwise adjustment is enough, then the COBRA-library may be planned as following:
a) select the desired "change of abundance" level for each locus;
b) combine loci with similar "change of abundance" levels into groups and choose a common level for each group;
c) select for each group of loci a library preparation protocol with the required "change of abundance" level.

Stepwise regulation of abundance allows reducing the dynamic range of concentrations to some acceptable level. If to combine transcripts in three groups: "without suppresion", "10x suppression" and "100x suppression," according to their expression level, the dynamic range would be reduced from five to three orders of magnitude (Figure 3).

Locus-specific oligonucleotides corresponding to different adjustment levels (and participating in different protocols) should be somehow grouped. This can be done in two ways:
- by spatial isolation: oligonucleotides may be assembled in separate tubes according to adjustment levels;
- by labeling: oligonucleotides from different groups may be combined together, if group-specific markers are introduced into oligonucleotides.

***Spatial isolation*** of locus-specific oligonucleotides enables performing of spatially isolated reactions. Library preparation reactions correspondent to different adjustment level groups may be completely independent from each other or differing only by a certain stage. Independent preparation of libraries for loci with different adjustment level gives a full freedom in choosing the protocol (different principles, different enzymes), but requires more labor and can lead to unstable results of comparison of the expression levels of genes from different adjustment level groups. Minimizing the number of differing stages decreases labor costs and makes the comparison of expression levels of different transcripts more reproducible. A spatially separated stage can be introduced at any point of the library preparation protocol:
- in the beginning - for example, separation of the original mixture (Examples 3, 4);
- in the middle part of protocol - for example, different conditions of preamplification of library molecules belonging to different adjustment level groups (Example 6);
- at the end - for example, classical RNA-Seq reaction, followed by separate hybridization-based selection with oligonucleotides belonging to different adjustment level groups (Example 7).
The reaction conditions would be as similar as possible, if primers for different groups are added subsequently to the same reaction (Examples 5 and 6).

***Markers of abundance level correction*** introduced in the locus-specific oligonucleotides allow to minimize differences in the reaction conditions and even to synthesize a sequencing library without separation of the mixture. There are a variety of experimental realizations of using marker regions for abundance level correction, from primitive like "divide the mixture into fractions by hybridization and then take the appropriate part of the volume of each fraction", to sophisticated like marker-specific PCR with different number of cycles for different markers (see the next section).

One preferred embodiment of the present invention refers to methods, wherein the relative abundance of components under analysis is changed in step iii) by using different reactions for different locus-specific oligonucleotides.Thereby it is preferred that differences in reactions are selected from the group comprising or consisting of: different amounts of original mixture containing nucleic acids used in reactions; different number of cycles in cyclic amplification reactions; different reaction times in linear amplification reactions.

Stepwise abundance level correction has certain advantages and disadvantages. Advantages:
- convenient approach for wide range regulation of abundance;
- modified primers are not required (if compare with using of "functional/blocked" primers", see later);
- any degree of suppression / enhancement can be reached and accurately reproduced. For example: using serial dilutions it is possible to take accurately 1/10000 of the reaction mixture; 10 cycles of PCR amplification give a quite accurate enhancement in 1000 times;
   adjustment level for the group as a whole can be easily changed.
Disadvantages:
- stepwise regulation and the number of steps is limited;
- reactions with different groups of primers are performed separately which reduces the reliability of the method and makes it dependent on the precision / accuracy of the separation of the mixture.
- transferring a locus from one group to another requires regrouping of oligonucleotides.

### Functional and blocked primers

One preferred embodiment of the present invention are methods wherein the locus-specific oligonucleotides of step iii) contain a mixture of functional and blocked oligonucleotides, wherein the concentration ratio of functional to blocked oligonucleotides is equivalent to the predefined value of change of abundance of this locus. Thereby it is further preferred that blocked oligonucleotides cannot be elongated in such reactions as primer extension, first-strand synthesis, second-strand synthesis, PCR, or gap-filling because they have 3' end modification. Another preferred aspect of the present invention are methods wherein blocked oligonucleotides cannot participate in ligation in such reactions as ligation detection reaction, gap-filling, LCR, or DANSR because they have 3' or 5' end modification.

Figure 4 shows how the mixture of functional and blocked primers allows adjusting abundance individually and independently for each locus. Functional and blocked primers may be designed in many ways. Blocked oligonucleotides should compete with "functional" oligonucleotides in the same reaction, but blocked oligonucleotides either block the reaction, or the reaction products can be separated from the reaction products obtained from functional oligonucleotides. In fact, blocked oligonucleotides suppress the reaction, and the degree of suppression is determined by the ratio of concentrations functional to blocked oligonucleotides.

Ratio of "functional/blocked" oligonucleotides can be selected independently for each locus. As a result, the efficiency of conversion of original molecules into molecules of the library can be tuned independently for each locus.

Different blocking approaches may be used for primer-dependent reactions:
- primer extension: blocking of 3' end of primers (e.g. 3' amino-modified primer);
- ligation: blocking of 3' end of upstream primers (e.g. 3' amino-modified primer); blocking of 5' end of downstream primers (e.g. 5' dephosphorilated primer);
- hybridization-based selection: using primers without a complementary region (e.g. region for hybridization or for PCR-amplification);
- affinity selection: using primers without affinity region (e.g. biotinilated/nonbiotinilated molecules).

One further aspect of the present invention relates to methods wherein functional and/or blocked oligonucleotides have markers, allowing separating hybrids of functional oligonucleotides with target or products of reaction involving functional oligonucleotides from the hybrids of blocked oligonucleotides with target or products of reaction involving blocked oligonucleotides.

Functional and blocked primers producing separable reaction products, allow to work both with suppressed (after removal of the sequencing library molecules synthesized by using the "blocked" primers), and with non-suppressed library (without separation of the sequencing library molecules synthesized by using the "blocked" primers). Besides, enzymatic reactions are provided with a high concentration of substrate at all stages of library preparation (some enzymes do not work well with the substrate at low concentrations).

Different approaches allow to separate the reaction products obtained from functional and blocked oligonucleotides:
- biotinylated "functional" or "blocked" primers - corresponding library molecules can be attached to streptavidin-coated particles;
- "blocked" primers containing deoxyuridine - corresponding library molecules can be destroyed by UDGase. Similarly, methylated "functional" and unmethylated "blocked" primers - only unmethylated products could be digested by methylation-sensitive restriction enzymes;
- "functional" primers with a conservative 5' region (for further amplification with common primers). "Blocked" primers would not contain this region.

Therefore within the present invention it is preferred that the markers are selected from the group comprising:
- dUTP used instead of dTTP;
- thio-modified oligonucleotides;
- oligonucleotides with biotin;
- oligonucleotides with 5-bromo-2'-deoxyuridine (BrdU);
- presence in oligonucleotides of constant parts for subsequent amplification or hybridization-based selection.

Advantages of COBRA methods based on using a mixture of "functional / blocked" primers:
- independent suppression level regulation for each individual locus;
- change of the regulation level does not require synthesis of new oligonucleotides;
- library preparation reactions are performed in one mixture in the same conditions;

Disadvantages:
- except for the usual set of "functional" primers, at least one additional "blocked" primer is required;
- change of the regulation level requires redesign of the oligonucleotides mixture.

Three mentioned approaches to the implementation of COBRA are simple and intuitively understandable:
- different number of loci per transcript;
- different library-preparation protocols for different abundance correction groups;
- using modified locus-specific oligonucleotides that compete with normal locus-specific oligonucleotides, but do not produce library molecules.
Using these approaches almost any existing method of sequencing libraries preparation can be adapted to COBRA.

COBRA is useful if components of nucleic mixture have different representation: for transcriptome analysis (mRNA, small RNA's), for analysis of biodiversity in environmental or clinical samples, etc.

Since the suppression level for individual components is known and is accurately reproduced, the concentrations of components may be measured precisely. To determine concentrations of components in the original NA mixture their concentrations in the derivative mixture should be multiplied on corresponding suppression levels. It allows to compare not only the pairs of experiments between each other, but also the experiments performed by different people in different laboratories using different COBRA-based protocols.

It is possible to evaluate the expression level of ribosomal RNA using COBRA approach. Currently, this assessment is not conducted, since the level of ribosomal RNA is too large and researchers try to exclude ribosomal RNA from the analysis completely. COBRA gives a possibility to reduce concentration of ribosomal RNA to an acceptable level and thus to analyze its expression.

The COBRA procedure requires locus-specific oligonucleotides, but it is not a big inconvenience for performing routine analyses. For example, in clinical analysis only a few types of human tissues are easily available (blood, saliva, buccal cells, sperm, feces). For each of these tissues, appropriate locus-specific oligonucleotides can be designed.

COBRA approaches with a positive selection (wherein sequencing library contains only components corresponding to locus-specific oligonucleotides i.e. all other nucleic acid components of original mixture are removed by creation of a sequencing library) allows to analyze only a predefined set of transcripts. In some cases this is advantageous:
- positive selection is well suited for clinical testing and routine analysis of biodiversity, for which the hypothesis-free possibilities usually remain unclaimed;
- there is no need in ribosomal RNA depletion; this is especially convenient for the analysis of bacterial transcription, where polyA⁺ selection cannot be applied;

Despite using of locus-specific oligonucleotides COBRA approach may be hypothesis-free and allow to analyse all components of the mixture (Example 12). For that not positive but negative COBRA selection should be performed, where locus-specific primers are used to reduce the number of transcripts which otherwise get oversequenced and wherein sequencing library preserves all components which have no corresponding locus-specific oligonucleotides. Negative COBRA selection has the following advantages:
- it is a hypothesis-free approach;
- if the negative selection is applied for the change of composition of starting material, the procedure is easily compatible with any sequencing library preparation protocol;
- the procedure can be combined with the removal of ribosomal RNA;

One preferred aspect of the present invention are methods, wherein subsequent nucleic acid mixture contains only components corresponding to locus-specific oligonucleotides i.e. all other nucleic acid components of original mixture are removed by creation subsequent nucleic acid mixture or wherein subsequent nucleic acid mixture preserves all components which have no corresponding locus-specific oligonucleotides.

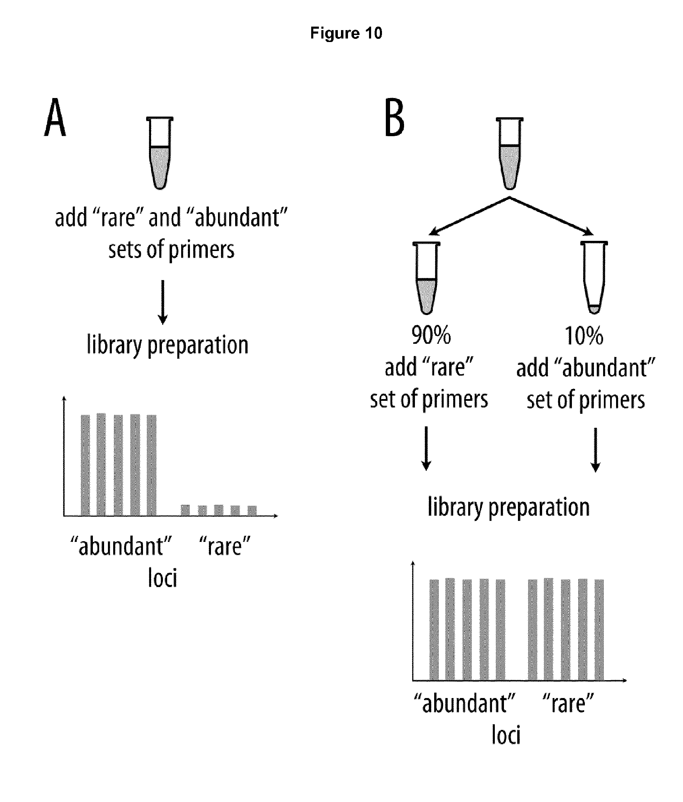

It is possible to use a combination of methods. Figure 10 shows an example of combination of "abundance correction groups" and "functional / blocked primers" approaches. Let's assume that there is a kit for preparation of COBRA RNA-Seq libraries. Locus-specific primers in the kit are divided into two sets: "abundant" and "rare". When using all primers in the common reaction, the number of clones per locus is about 10 times higher for the abundant group, if compare with the rare group. This is useful, because otherwise for the limited amount of starting material low fidelity results would be obtained both for the abundant and for the rare loci. However, when there is an excess of starting material and the results for the rare group are quite reliable, it is not practical to maintain a ten-fold excess for the abundant group. Then it makes sense to use the abundant set of primers for the synthesis of libraries with less starting material to level off the representation.

The present invention refers further to a kit, suitable for analysis of concentrations of nucleic acid according to any one of claims 1 - 16, which allows to produce from original mixture containing nucleic acids some subsequent nucleic acid mixture, wherein abundance of definite set of components is decreased in predefined manner in comparison with abundance of at least one other component.

### Examples

**Example 1.** Preparation of the sequencing library by ligation of detector oligonucleotides on a cDNA template.

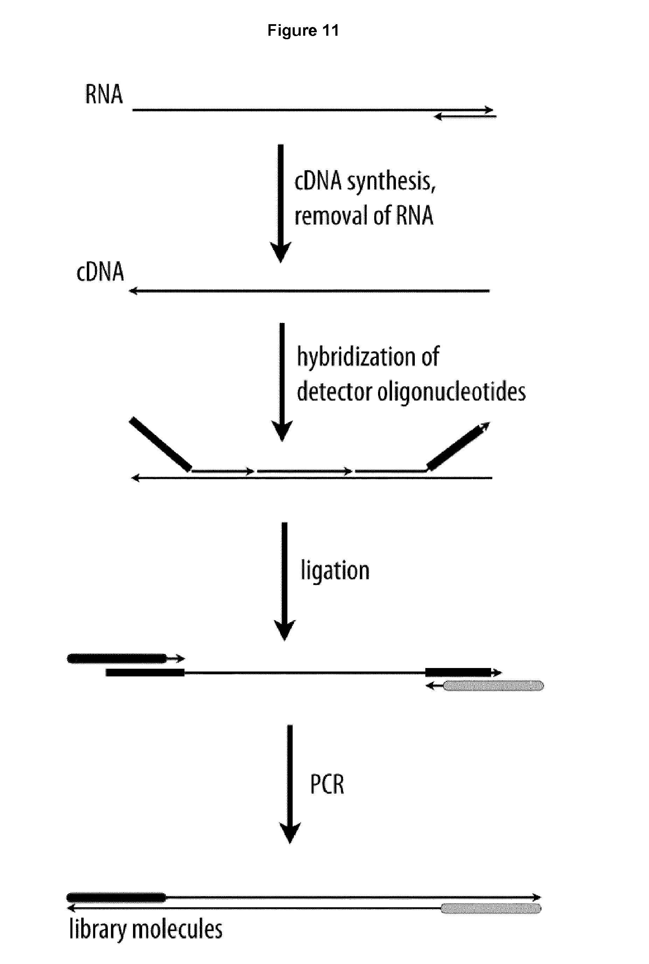

The scheme of the sequencing library preparation is shown in Figure 11. The protocol is adapted from [2]. After cDNA synthesis and RNA removal, selected loci are detected by cDNA-dependent ligation of locus-specific detector oligonucleotides.

Three detector oligonucleotides are used for each locus. Flanking oligonucleotides contain regions corresponding to the sequencing library adapters: 5'-region of the upstream oligonucleotide and 3'-region of the downstream oligonucleotide.

Following ligation, and getting rid of most of the non-ligated oligonucleotides the library amplification is performed. During amplification ligated molecules acquire full-size sequencing adapters.

Sequencing is used for detection, accounting and quality control of library molecules. If a sequenced molecule contains fragments belonging to different loci or fragments are ligated in the wrong order, it is excluded from the further analysis.

**Example 2.** Preparation of the sequencing library by ligation of detector oligonucleotides on a RNA template.

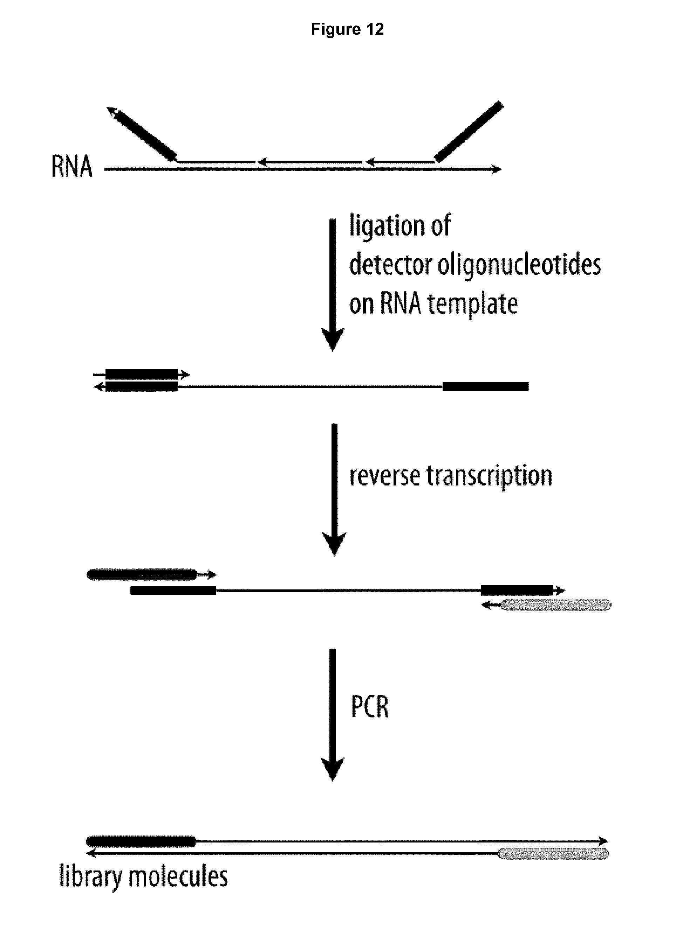

T4Rnl2 RNA ligase enzyme can be used for ligation of detector oligonucleotides directly on the RNA template [3]. Figure 12 shows the scheme of the corresponding protocol. For efficient ligation it is necessary that at least 3'-regions of upstream and middle oligonucleotides consist of ribonucleotides. Library molecules are obtained after reverse transcription of the ligated oligonucleotides.

Following ligation, getting rid of most of the non-ligated oligonucleotides and reverse transcription the library amplification is performed. During amplification ligated molecules acquire full-size sequencing adapters.

Sequencing is used for detection, accounting and quality control of library molecules. If a sequenced molecule contains fragments belonging to different loci or fragments are ligated in the wrong order, it is excluded from the further analysis.

**Example 3.** Separate reactions for different groups of detector oligonucleotides

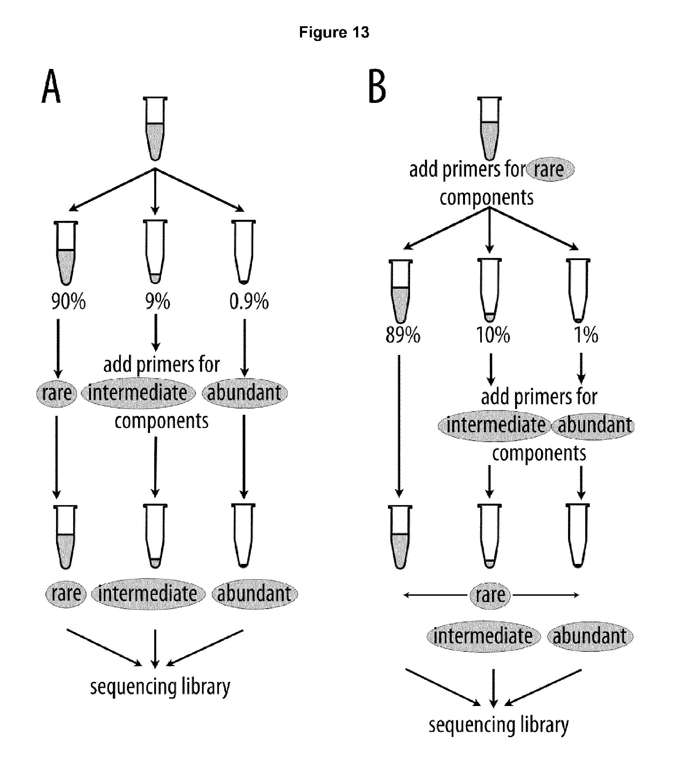

Genes with "high", "intermediate" and "low" levels of expression were selected, 10 genes in each group. Using the procedure described in Example 1 two sequencing libraries were prepared. When preparing the first library primers for all loci were used together. For the preparation of the second library reaction mixture was divided into three separate reactions, as shown in Figure 13A.

It was found that the frequency of sequencing reads corresponding to genes with a "high" and "intermediate" levels of expression is reduced in the second library in 100 and 10 times respectively.

**Example 4.** Separate reactions for different groups of detector oligonucleotides

COBRA library was prepared using the same primers as in the Example 3, but the reaction mixture was divided, as shown in Figure13B.

In Example 3 some unwanted suppression occurs, since ∼10% of the starting material is inaccessible to the primers corresponding to low expressed genes. On the scheme shown in Figure 13B, suppressed are only those genes that really need to be suppressed.

**Example 5**. Different number of ligation cycles for different groups of primers

When using a thermostable ligase (e.g. Pfu or Taq ligase) detection reaction described in the Example 1 can be performed cyclically, each cycle consisting of steps of denaturation, annealing and ligation. This allows to obtain several library molecules from each template cDNA.

It is possible to change the relative abundance of the library molecules corresponding to different adjustment level groups, if corresponding groups of locus-specific detector oligonucleotides are introduced into a cyclic ligase reaction after different numbers of cycles. The earlier detector oligonucleotides are introduced into the cyclic ligase reaction, the more library molecules would be obtained from each template cDNA.

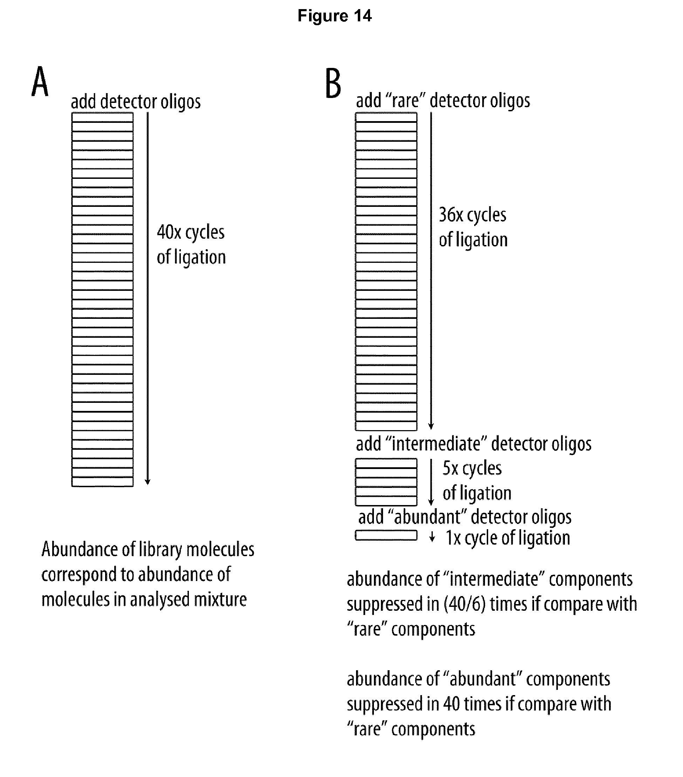

On the scheme shown in Figure 14 relative concentrations of abundant and intermediate groups of loci fell 40 and 6,7 times respectively due to the different number of ligation cycles for different groups of primers:
- 40 for primers corresponding to rare loci;
- 6 for primers corresponding to intermediate loci;
- 1 for primers corresponding to abundant loci.

**Example 6.** Different number of amplification cycles for different groups of primers

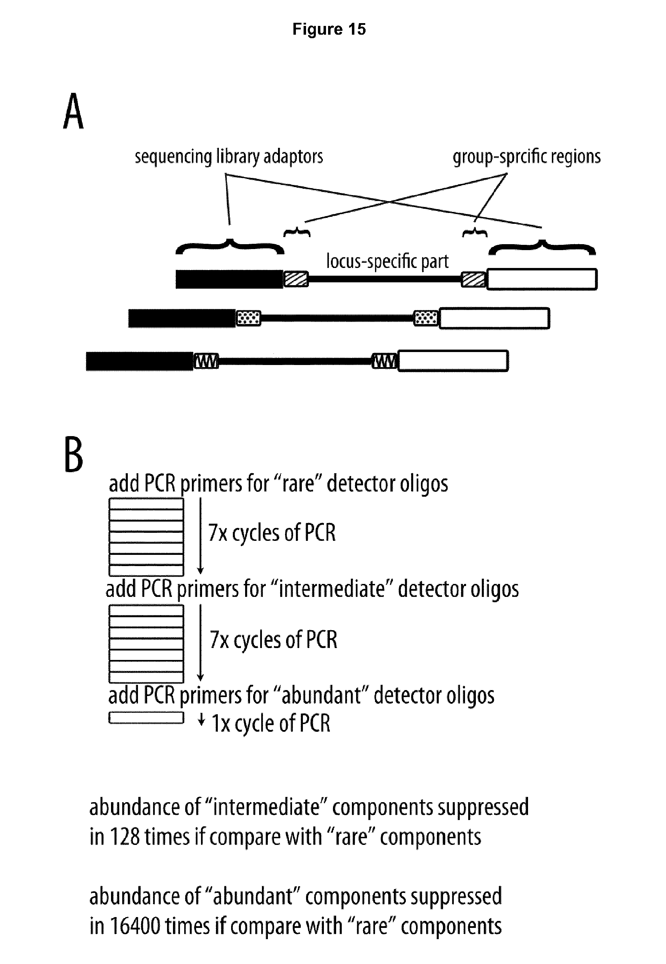

If oligonucleotides used in the reaction described in Example 1 have the structure shown in Figure 15A, a stepwise change of relative concentrations of different groups of transcripts can be carried out at the stage of library preamplification. To provide group-specific amplification 3' ends of group-specific PCR-primers should correspond to group-specific regions of ligated detector oligonucleotides.

As in the previous example, group-specific PCR-primers should be added on different PCR cycles (Figure 15B). Marker region would provide selective amplification of specific library molecules.

On the scheme shown in Figure 15 relative concentrations of abundant and intermediate groups of loci fell 16400 and 128 times respectively due to the different number of cycles of PCR for different groups of primers:
- 15 for primers corresponding to rare loci;
- 8 for primers corresponding to intermediate loci;
- 1 for primers corresponding to abundant loci.

Examples 5 and 6 show how stepwise level adjustment can be carried out in a common reaction mixture. In Example 5, spatial isolation of oligonucleotides from different adjustment level groups is used, and in Example 6 oligonucleotides of different adjustment level groups have different markers.

**Example 7.** Stepwise COBRA selection of RNA-Seq library molecules.

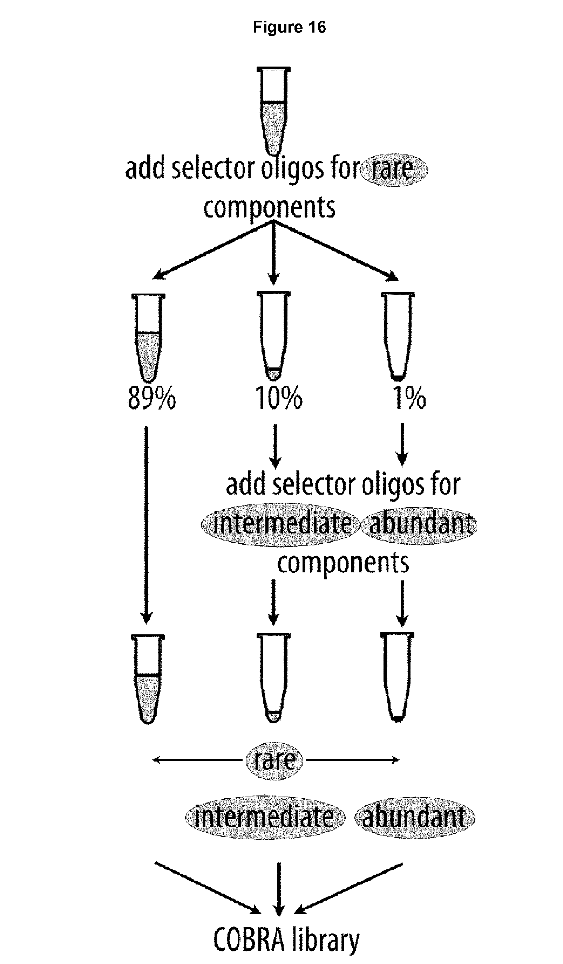

COBRA changing of abundance can be carried out directly prior to sequencing of a standard RNA-Seq library. The scheme is shown in Figure 16. Hybridization with biotinylated locus-specific selector oligonucleotides is performed to fish out transcripts of interest from RNA-seq library.

Library is divided into portions. For each portion selector primers belonging to groups with corresponding adjustment levels are applied. Relative abundance of different transcripts is changed because only a certain part of the library is available to selector oligonucleotides from a particular adjustment level group.

Performing COBRA-procedure prior to sequencing is convenient because:
- the procedure can be easily adapted to different protocols of RNA-Seq library preparation;
- only one selector oligonucleotide per locus is required;
- when selector oligonucleotide is long enough, procedure is not sensitive to point mutations located in the hybridizing region;
- for standard applications standard sets of selector oligonucleotides can be used. For example, in the routine clinical analysis only a few types of human tissues are easily available (blood, saliva, buccal cells, sperm, feces). For each of these tissues, appropriate COBRA selector oligonucleotides can be designed.

**Example 8**. Functional/blocked primers: arrested primer extension

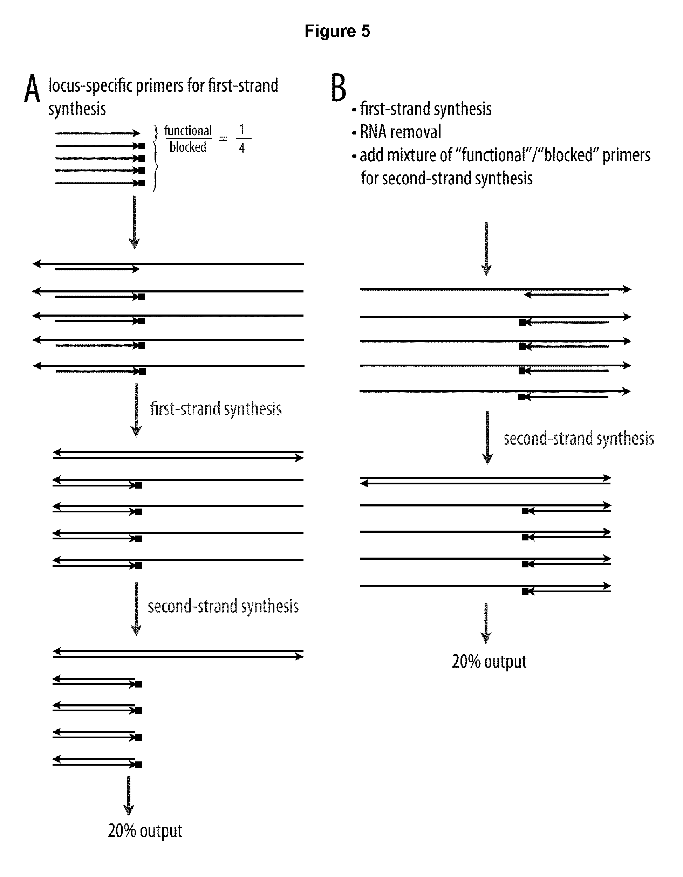

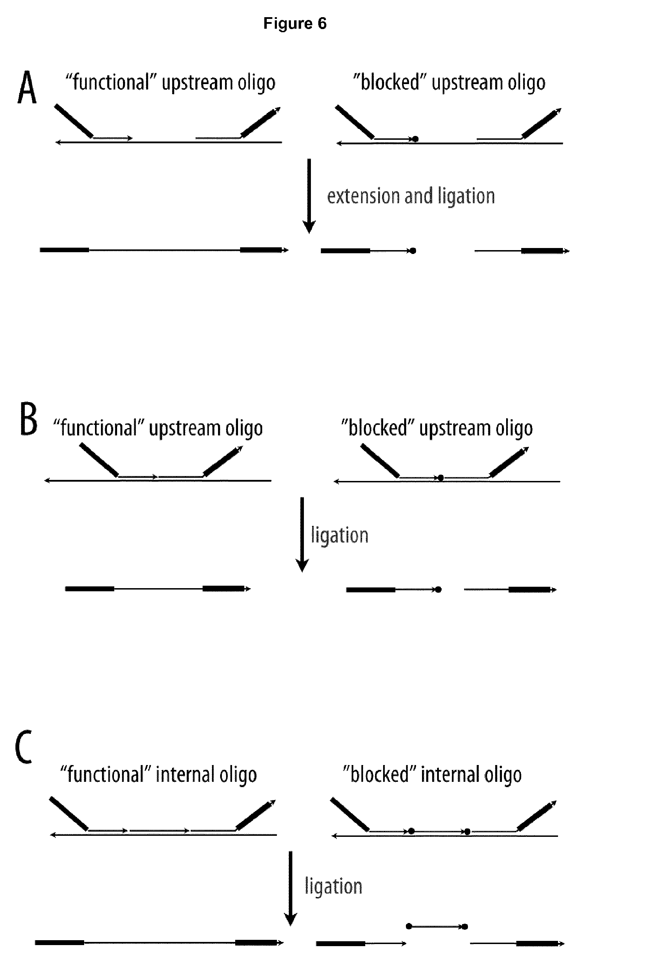

Examples of using blocked primers which are unable to participate in primer extension reaction are shown in Figures 5A, 5B and 6A.

Fig. 5A shows the protocol for preparation of a COBRA RNA-Seq library with a partial blocking of the first strand synthesis. Among the advantages of the method is the small number of primers (one per locus), which however can cause high background. Obtained library molecules are heterogeneous, which can be inconvenient for the analysis of the sequencing data.

To reduce the number of molecules of the library, synthesized from non-specific primers, it makes sense to use primers with the 5' part correspondent to the sequencing adapter. Then during the preparation of the library, only the second sequencing adapter should be ligated.

Fig. 5B shows a protocol with blocked synthesis of the second strand. If 5' parts of the primers used for first- and second-strand synthesis are conservative and correspond to sequencing adapters, library molecules are obtained immediately after synthesis of the second strand.

Fig. 6A shows a scheme of gap-filling reaction. This approach is useful to analyze polymorphic regions. If "blocked" primer can't be extended in the course of primer extension reaction, a gap between the detector oligonucleotides would remain, and ligation would not occur.

**Example 9.** Functional/blocked primers: arrested primer ligation

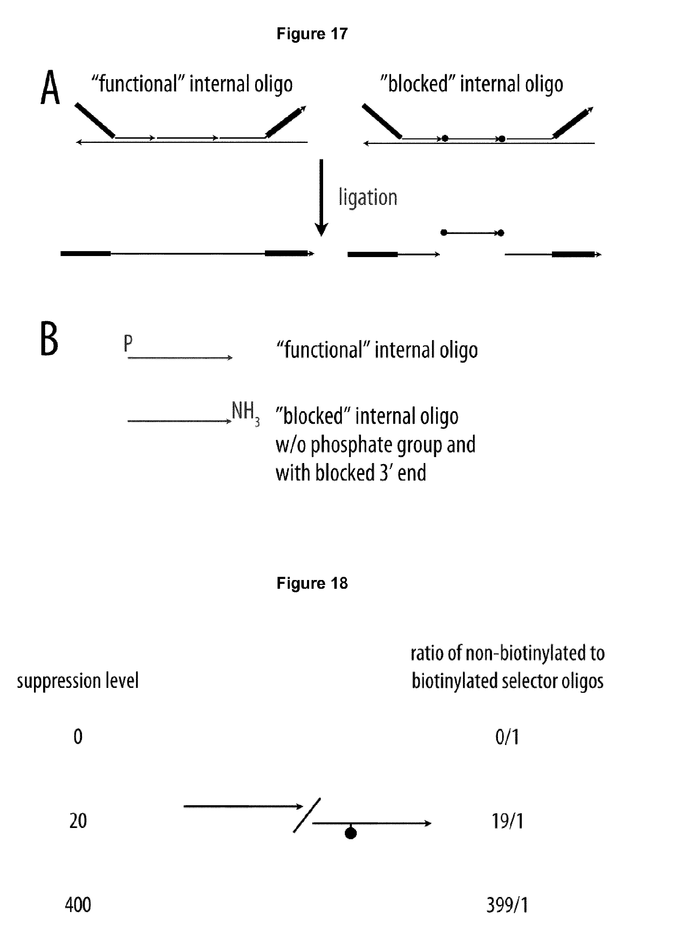

Examples of using blocked primers which are unable to participate in ligation reaction are shown in Figures 6B, 6C and 17.

The use of two (or three) specific primers for each locus reduces the number of non-specific molecules in the library.

If it is necessary to analyze a polymorphic region, the ligation can be combined with gap-filling reaction (Figure 6A).

If 5'- parts of the upstream and 3'-parts of downstream primers are conservative and correspond to sequencing adapters, library molecules are obtained immediately after ligation.

### Using functional/blocked primers for preparation of COBRA library

COBRA library was made according to the protocol described in Example 1 using a mixture of functional/blocked primers (Fig. 17). The structure of blocked primers is shown in Fig. 17B. For primers corresponding to genes with "high", "intermediate" and "low" levels of expression the ratio of functional/blocked primers was "1 :99", "1 :9" and "1:0", respectively.

It was found out that the frequency of sequencing reads corresponding to genes with "high" and "intermediate" level of expression is reduced in 100 and 10 times respectively.

**Example 10.** Selectable blocked and functional library molecules

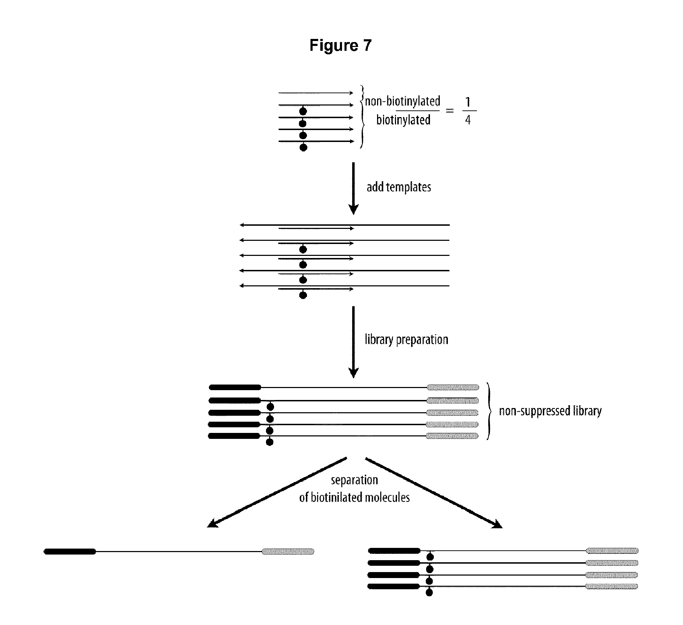

Schemes of methods that allow to separate the molecules produced in the reaction with the participation of functional primers from the molecules derived from reactions with blocked primers are shown in Figures 7,8,9.

Fig. 7 shows a protocol where "blocked" primers are biotinylated - corresponding library molecules can be bound to streptavidin coated particles and excluded from sequencing.

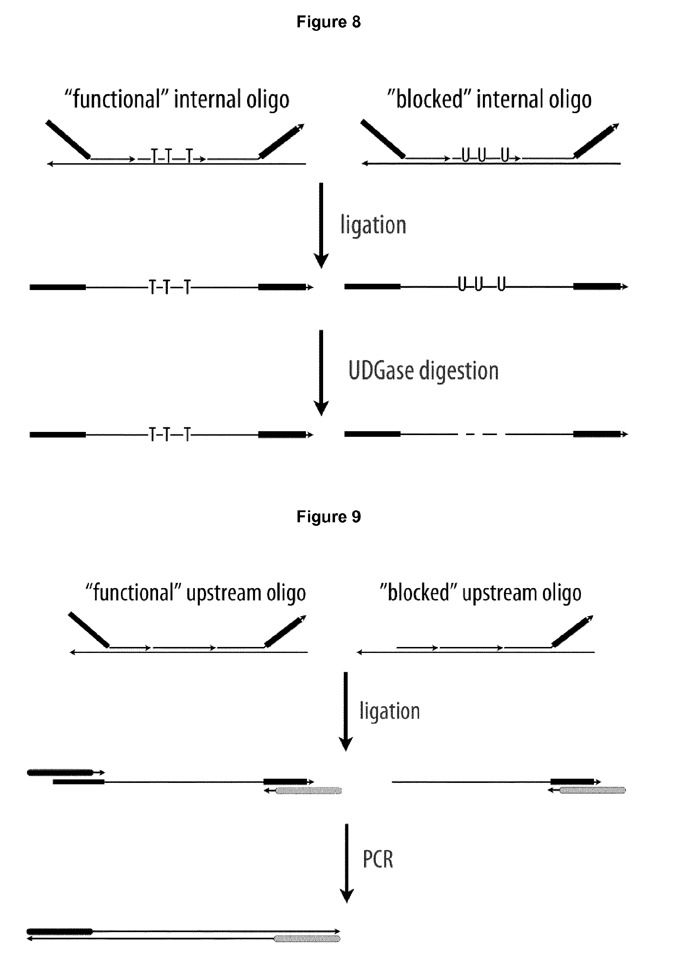

Fig. 8 shows a protocol where blocked primers contain uridine - corresponding library molecules can be destroyed by UDGase. Library molecules originating from the "functional" primers withstand UDGase treatment.

Fig. 9 shows the protocol where functional upstream detector oligonucleotides contain a conservative 5' region (for further amplification of library molecules). Blocked upstream detector oligonucleotides do not contain such a region. After ligation, amplification is carried out using primers corresponding to conservative regions. Library molecules originating from the functional primers are amplified, and, besides acquire full-size sequencing adapters.

**Example 11**. Functional/blocked primers: COBRA selection of RNA-Seq library molecules.

The use of functional / blocked oligonucleotides allows to perform COBRA-selection of RNA-Seq library molecules before sequencing without splitting the reaction mixture into portions (as in Example 7). Sets of "functional" and "blocked" selector oligonucleotides for different suppression levels are shown in Figure 18. "Functional" selector oligonucleotides are biotinylated and library molecules hybridized to them can be fished out and sequenced. Blocked selector oligonucleotides are not biotinylated: they do not allow to fish out library molecules and prevent binding of library molecules to biotinylated selector oligonucleotides. Proportion of molecules selected for sequencing is determined individually for each locus by the ratio of concentrations of locus-specific biotinylated and non-biotinylated oligonucleotides.

**Example 12.** Functional/blocked primers: negative COBRA-selection of RNA molecules for preparation of sequencing library

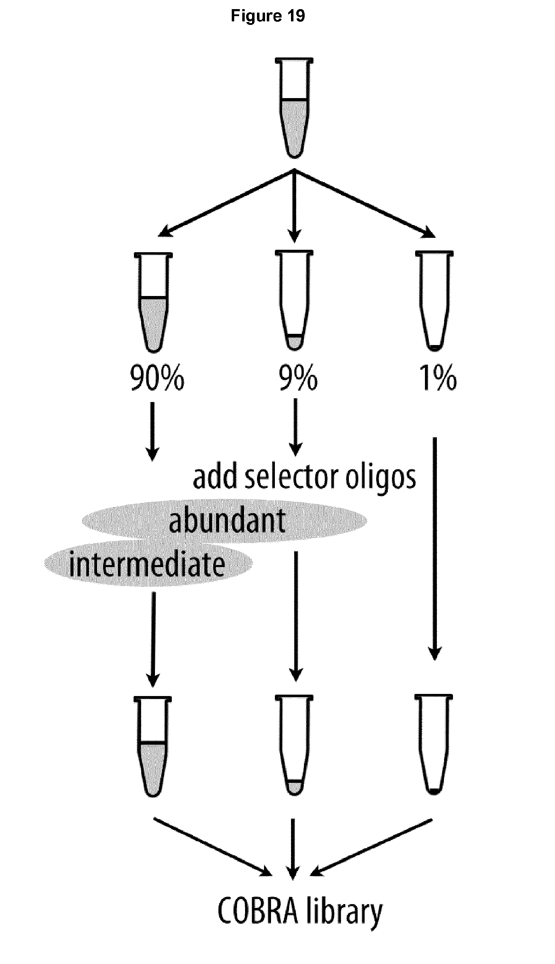

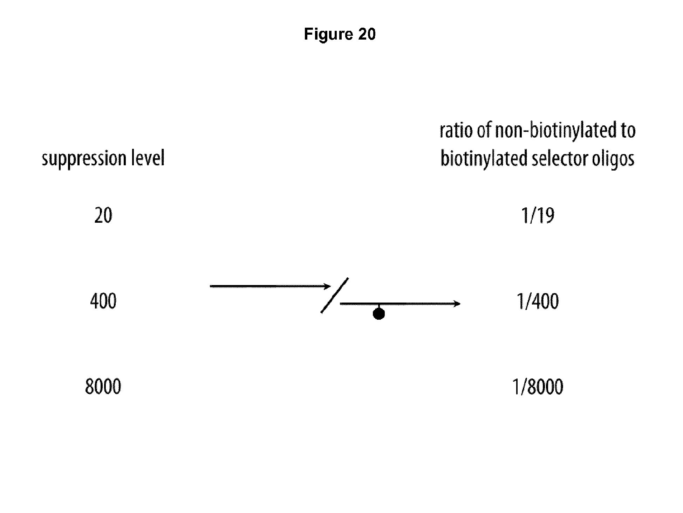

Figures 19 and 20 show the implementation of hypothesis-free COBRA procedures using stepwise abundance adjustment (Figure 19) and using "functional / blocked" selector oligonucleotides for abundance adjustment individually for each locus (Figure 20). Hypothesis-free COBRA approach is based on the removal of a certain part of transcripts, which otherwise get oversequenced.

For stepwise level adjustment original mixture is divided into portions and selector oligonucleotides corresponding to different adjustment levels are added to the portions, as shown in Figure 19. Since for each adjustment level group some part of the mixture remains inaccessible to selector oligonucleotides, a certain portion of transcripts remains for the analysis.

Negative selection can be performed in a single tube without division into portions, if functional / blocked selector oligonucleotides are used (Figure 20). Performing selection in one tube reduces handwork and makes comparison of the concentrations of various transcripts more reliable. Functional selector oligonucleotides are not biotinylated, they prevent hybridization of the transcripts with biotinylated selector oligonucleotides. Which portion of transcripts remains in the mix for later analysis is determined individually for each locus by the concentration ratio of locus-specific biotinylated and non-biotinylated oligonucleotides.

### References

1. Bullard DR, Bowater RP. Direct comparison of nick-joining activity of the nucleic acid ligases from bacteriophage T4. Biochem J. 2006 Aug 15;398(1):135-44.
2. Christodoulou DC, Gorham JM, Herman DS, Seidman JG. Construction of normalized RNA-seq libraries for next-generation sequencing using the crab duplex-specific nuclease. Curr Protoc Mol Biol. 2011 Apr;Chapter 4:Unit4.12.
3. Sparks AB, Wang ET, Struble CA, Barrett W, Stokowski R, McBride C, Zahn J, Lee K, Shen N, Doshi J, Sun M, Garrison J, Sandler J, Hollemon D, Pattee P, Tomita-Mitchell A, Mitchell M, Stuelpnagel J, Song K, Oliphant A. Selective analysis of cell-free DNA in maternal blood for evaluation of fetal trisomy. Prenat Diagn. 2012 Jan;32(1):3-9. doi: 10.1002/pd.2922. Epub 2012 Jan 6.

### Description of the Figures

**Figure 1****: COBRA approach. A.** Traditional sequencing library. The abundance of cDNA fragments matches the abundance of transcript in the analyzed mixture. **B.** COBRA approach. The abundances of molecules in COBRA sequencing library are adjusted according to the required accuracy of concentration measurement. Suppression levels for each component are shown on the graph. Concentrations of components in the analyzed mixture may be determined by multiplying concentrations in the COBRA-library on corresponding suppression levels.
**Figure 2****: Different number of detectable loci for different components of nucleic acid mixture**. Contour arrows show components of nucleic acid mixture "α" and "β" which have different concentration. Solid arrows correspond to locus-specific oligonucleotides used for preparation of sequencing library. **A.** In case there is one detector locus per each component the number of sequencing reads corresponding to components "α" and "β" considerably differs. **B.** If more detector loci are selected for the rare component, the number of sequencing reads corresponding to components "α" and "β" is comparable.
**Figure 3****: Stepwise decrease of dynamic range of concentrations**. Components of the mixture with dynamic range of concentrations of five orders of magnitude are assigned to three groups according to their level of abundance (shown in different colors). COBRA-sequencing libraries are prepared using three different library preparation protocols "without suppresion", "10x suppression" and "100x suppression". Dynamic range of concentrations of COBRA library molecules is three orders of magnitude.
**Figure 4****: Abundance adjustment using a mixture of functional and blocked primers**. Locus "α": all primers are functional, no suppression occurs. Locus "β": a mixture of functional and blocked primers (1:4). Because of competition for the template, the yield of library molecules will decrease in 5 times.
**Figure 5****: Schemes of cDNA synthesis methods using functional and blocked primers in primer extension reaction. A**. 80% blocking of the first strand synthesis. Locus-specific oligonucleotides (functional/blocked = 1:4) are used for cDNA synthesis. **B**. 80% blocking of the second strand synthesis. Locus-specific oligonucleotides (functional/blocked = 1:4) are used for initiation of second strand synthesis. In both cases only fifth part of transcripts results in corresponding ds cDNA molecules.
**Figure 6****: Schemes of methods using functional and blocked primers. A.** Gap-filling. **B**. Allele-specific ligation C. DANSR.
**Figure 7****: Using of biotinilated primers as blocked primers.** Sequencing library molecules are prepared using a mixture of biotinilated and non-biotinilated (4:1) locus-specific oligonucleotides. As a result the fifth part of library molecules are not biotinilated. Before sequencing, biotinilated molecules are removed, and non-biotinilated are sequenced, providing a 80% suppression.
**Figure 8****: Using of dUTP-containing primers as blocked primers.** Sequencing library is prepared using sets of locus-specific oligonucleotides, three per locus. They need to be ligated to produce a library molecule. To regulate the representation of library molecules corresponding to a certain locus, a mixture of "functional" internal oligonucleotides (with standard nucleotides) and "blocked" (containing uridines in the T positions) is used. Both types of internal oligonucleotide participate in ligation, however library molecules with "blocked" oligonucleotide are destroyed by UDGase prior to sequencing. The ratio of standard oligonucleotide to the uridine-containing one determines the level of suppression.
**Figure 9****: Using primers with conservative 5' region as functional primers.** Sequencing library is prepared using sets of locus-specific oligonucleotides, three per locus. They need to be ligated and then amplified to produce a library molecule. To regulate the representation of library molecules corresponding to a certain locus, a mixture of functional upstream oligonucleotides (with conservative 5' region) and blocked (without conservative 5' region) is used. Both types of upstream oligonucleotide participate in ligation, however library molecules with blocked oligonucleotide don't have a binding region for the PCR primer and can't be amplified. The ratio of oligonucleotides with and without the 5' tail for amplification determines the level of suppression.
**Figure 10****: The use of different abundance regulation schemes under different conditions. A.** Limited amount of starting material. **B**. The amount of starting material is sufficient to obtain reliable data for the "rare" loci.
**Figure 11****: Scheme of digital analysis of selected regions (DANSR) [2].** For each locus of interest a set of three locus-specific oligonucleotides is used. They need to be ligated to produce a molecule with 5' and 3' regions correspondent to sequencing adapters.
**Figure 12****: Ligation of detector oligonucleotides on RNA template**. For each locus of interest a set of three locus-specific oligonucleotides is used. They need to be ligated to produce a molecule with 5' and 3' regions correspondent to sequencing adapters. Reverse transcription is done before the amplification.
**Figure 13****: Suppression of individual loci due to performing reaction with part of the original material. A.** Separation of original material before primers addition. **B**. Reaction scheme avoiding unwanted suppression of rare loci.
**Figure 14****: Different number of cycles in cyclic ligation reaction for different adjustment level groups of loci. A.** Standard scheme of cyclic ligation. All detector oligonucleotides are added in the beginning of cyclic ligation. **B**. COBRA cyclic ligation. Detector oligonucleotides corresponding to different adjustment level groups are introduced into a cyclic ligase reaction after different numbers of cycles.
**Figure 15****: Different number of PCR cycles for different adjustment level groups of loci. A.** Structure of ligated DANSR detector oligonucleotides for COBRA PCR amplification. Regions of flanked detector oligonucleotides correspondent to adjustment level groups are used for group-specific PCR. **B**. COBRA PCR amplification. PCR primers corresponding to different adjustment level groups are introduced into amplification reaction after different numbers of cycles.
**Figure 16****: Stepwise positive COBRA selection.** There are three groups of selector oligonucleotides: (i) "rare" group without adjustment level correction; (ii) "intermediate" group with 10x abundance suppression; (iii) "abundant" group with 100x abundance suppression. Analyzed NA mixture is divided into portions correspondent to the abundance suppression level. "Rare" group is added to the whole NA mixture. "Intermediate" group is added to the 10% of the NA mixture. "Abundant" group is added to the 1% of the NA mixture. Selector oligonucleotides bind to the correspondent library molecules. Selected molecules are combined together to prepare a COBRA-library.
**Figure 17****: Scheme of DANSR methods using functional and blocked primers. A.** To regulate the representation of library molecules corresponding to a certain locus, a mixture of functional and blocked internal oligonucleotides is used. If blocked oligonucleotide is annealed to the template, ligation would not occur. **B**. Structure of internal DANSR primers. 3' and 5' ends of "blocked" primer are modified to prevent ligation.
**Figure 18****: Positive COBRA selection with functional/blocked primers**. Abundance adjustment level may be individually selected for each locus. Functional selector oligonucleotides are biotinylated. Blocked selector oligonucleotides are not biotinylated.
**Figure 19****: Stepwise negative COBRA selection.** There are two groups of selector oligonucleotides: (i) "intermediate" group with 10x abundance suppression; (ii) "abundant" group with 100x abundance suppression. In contrast to Figure 16 there is no "rare" selector oligonucleotide group: all untargeted transcripts remain for the analysis. Analyzed NA mixture is divided into portions correspondent to the abundance suppression level. "Intermediate" group is added to the 90% of the NA mixture. "Abundant" group is added to the 99% of the NA mixture. Selector oligonucleotides bind to the correspondent RNA molecules. Selected molecules are removed from the analysed mixture. The result of the negative selection is a COBRA RNA mixture. Any sequencing procedure may be used for the analysis of this COBRA RNA mixture.
**Figure 20****: Negative COBRA selection with functional/blocked primers.** Abundance adjustment level may be individually selected for each locus. "Functional" selector oligonucleotides are not biotinylated. "Blocked" selector oligonucleotides are biotinylated.

## Claims

1. Method of analysis of concentrations of nucleic acid components in mixtures containing nucleic acids, comprising the following steps:
i) providing an original mixture containing nucleic acids;
ii) selection of at least one nucleic acid component of the original mixture for which the abundance should be changed in predefined manner;
iii) creation from original mixture containing nucleic acids a subsequent nucleic acid mixture wherein abundances of components corresponding to the components selected on step ii) are changed in predefined manner using locus-specific oligonucleotides for said components;
iv) analysis of concentrations of at least two components in the subsequent nucleic acid mixture for which relative abundances were changed in predefined manner compared with relative abundances of corresponding components in the original mixture containing nucleic acids.

2. The method according to claim 1 wherein components of the original mixture containing nucleic acids and the values of change of abundance are selected on step ii) in such a way, that the dynamic range of concentrations of components under analysis in the subsequent nucleic acid mixture is lower than the dynamic range of concentrations of components under analysis in the original mixture containing nucleic acids or abundances of components under analysis in the subsequent mixture are more suitable for the required accuracy of analysis of concentrations.

3. The method according to claim 1 or 2 wherein the subsequent nucleic acid mixture is selected from the group comprising: sequencing library, set of ligated detector oligonucleotides, set of fluorescently labeled molecules, or nucleic acids molecules selected from the original mixture.

4. The method according to any one of claims 1 - 3, wherein analysis of concentration of components in the subsequent nucleic acid mixture on step iv) is performed by sequencing, hybridization with microarray, RT-PCR, digital PCR, digital color-coded barcode technology.

5. The method according to any one of claims 1 - 4, wherein concentrations of components under analysis in original mixture containing nucleic acids are calculated using predefined values of change of abundance of components under analysis.

6. The method according to any one of claims 1 - 5, wherein the relative abundance of at least one component under analysis is changed in step iii) by selecting suitable number of loci and correspondent locus-specific oligonucleotides for this component.

7. The method according to any one of claims 1 - 6, wherein said locus-specific oligonucleotides of step iii) contain a mixture of functional and blocked oligonucleotides, wherein the concentration ratio of functional to blocked oligonucleotides is equivalent to the predefined value of change of abundance of this locus.

8. The method according to claim 7, wherein blocked oligonucleotides cannot be elongated in such reactions as primer extension, first-strand synthesis, second-strand synthesis, PCR, gap-filling because they have 3' end modification.

9. The method according to claim 7, wherein blocked oligonucleotides cannot participate in ligation in such reactions as ligation detection reaction, gap-filling, LCR, DANSR because they have 3' or 5' end modification.

10. The method according to claim 7, wherein functional and/or blocked oligonucleotides have markers, allowing separating hybrids of functional oligonucleotides with target or products of reaction involving functional oligonucleotides from the hybrids of blocked oligonucleotides with target or products of reaction involving blocked oligonucleotides.

11. The method according to claim 10, wherein the markers are selected from the group comprising:
• dUTP used instead of dTTP;
• thio-modified oligonucleotides;
• oligonucleotides with biotin;
• oligonucleotides with 5-bromo-2'-deoxyuridine (BrdU);
• presence in oligonucleotides of constant parts for subsequent amplification or hybridization-based selection.

12. The method according to any one of claims 1 - 11, wherein the relative abundance of components under analysis is changed in step iii) by using different reactions for different locus-specific oligonucleotides.

13. The method according to claim 12, wherein differences in reactions are selected from the group comprising: different amounts of original mixture containing nucleic acids used in reactions; different number of cycles in cyclic amplification reactions; different reaction times in linear amplification reactions.

14. The method according to any one of claims 1 - 13, wherein subsequent nucleic acid mixture contains only components corresponding to locus-specific oligonucleotides i.e. all other nucleic acid components of original mixture are removed by creation subsequent nucleic acid mixture or wherein subsequent nucleic acid mixture preserves all components which have no corresponding locus-specific oligonucleotides.

15. The method according to any one of claims 1 - 14, wherein the nucleic acid of the original mixture is selected for expression profiling from the group comprising: RNA, total RNA, mRNA, mtRNA, rRNA, tRNA, dsRNA, small RNA / micro RNA, cDNA; for analysis of biodiversity from the group comprising: RNA or DNA from environmental or clinical sample.

16. The method according to any one of claims 1 - 15 used for routine analyses of biodiversity or expression profiling in medicine, veterinary, agriculture, ecological studies.

17. Kit, suitable for analysis of concentrations of nucleic acid according to any one of claims 1 - 16, which allows to produce from original mixture containing nucleic acids some subsequent nucleic acid mixture, wherein abundance of definite set of components is decreased in predefined manner in comparison with abundance of at least one other component.
